# EUROPEAN PATENT APPLICATION

(11) **EP 3 335 702 A1**
(43) Date of publication of application: **20.06.2018**
(21) Application number: 16204720.3
(22) Date of filing: 16.12.2016
(51) Int. Cl.: A61K 9/20, A61K 31/4162, A61P 3/10

(54) **PHARMACEUTICAL COMPOSITIONS COMPRISING OMARIGLIPTIN**

(71) Applicant: Hexal AG, 83607 Holzkirchen (DE)
(72) Inventor: BORN, Max, D-83607 Holzkirchen (DE); FISCHER-DAUT, Diana, D-83607 Holzkirchen (DE); PUZIK, Andreas, D-83607 Holzkirchen (DE)
(74) Representative: Kluschanzoff, Harald

(57) **Abstract**

The present invention relates to a pharmaceutical composition comprising omarigliptin and at least one pharmaceutically acceptable excipient, wherein the omarigliptin is in the form of a pharmaceutically acceptable acid addition salt and its use in the treatment of type-2 diabetes mellitus.

## Description

### FIELD OF THE INVENTION

The present invention relates to a pharmaceutical composition comprising omarigliptin and at least one pharmaceutically acceptable excipient, wherein the omarigliptin is in the form of a pharmaceutically acceptable acid addition salt and its use in the treatment of type-2 diabetes mellitus.

### BACKGROUND OF THE INVENTION

Omarigliptin (2R,3S,5R)-2-(2,5-difluorophenyl)-5-[2-(methylsulfonyl)-2,6-dihydropyrrolo[3,4-c]pyrazol-5(4H)-yl]tetrahydro-2H-pyran-3-amine acts as a dipeptidyl peptidase IV (DPP IV) inhibitor and is used for the treatment of type 2 diabetes mellitus.

WO 2010/056708 describes omarigliptin and a pharmaceutical composition comprising omarigliptin, microcrystalline cellulose, croscarmellose sodium, and magnesium stearate. Omarigliptin, microcrystalline cellulose and croscarmellose are blended first. The mixture is then lubricated by magnesium stearate and pressed into tablets. Stability of the formulation has not been tested.

WO 2015/031228 describes a stable pharmaceutical composition comprising omarigliptin and neutral excipients in an amount of at least 75% by weight of the pharmaceutical formulation, wherein the neutral excipients comprise at least one non-reducible sugar diluent. The diluent is mannitol or microcrystalline cellulose or a mixture of mannitol and microcrystalline cellulose. Exemplified pharmaceutical formulations are tablets and consist essentially of omarigliptin, mannitol, microcrystalline cellulose, croscarmellose sodium, and magnesium stearate. The tablets are prepared by wet or dry processing methods. Diluents that should not be included in the pharmaceutical formulations described in WO 2015/031228 include lactose monohydrate, polyvinyl pyrrolidone, silicified microcrystalline cellulose, hydroxyethyl cellulose, anhydrous lactose, mannose, glucose, maltose, other reducing sugars, Neusilin (aluminum magnesium metasilicate), Kollidon (polyvinyl pyrrolidone), crospovidone and those with acid functional groups or basic functional groups. It is disclosed that one concern with developing a pharmaceutical composition of omarigliptin is the chemical stability of the compound. Omarigliptin is said to be sensitive to oxidation, hydrolysis (acid and base catalysed) and Maillard degradation (browning) with reducible sugar impurities in excipients.

### SUMMARY OF THE INVENTION

It has surprisingly been found that by using omarigliptin in the form of a pharmaceutically acceptable acid addition salt, the Maillard reaction can be slowed down, therefore reducing the amount of impurities that form over time and leading to more stable pharmaceutical compositions. Without being bound by theory, it can be postulated that the kinetics of the degradation reaction (Maillard) are slowed down via protonation of the primary amine in omarigliptin.

Accordingly, the pharmaceutical composition of the present invention comprises omarigliptin and at least one pharmaceutically acceptable excipient, wherein the omarigliptin is in the form of a pharmaceutically acceptable acid addition salt.

Even though omarigliptin is known to be prone to degradation in the presence of reducible sugar impurities in excipients, the use of omarigliptin in the form of a pharmaceutically acceptable acid addition salt allows for the use of reducible sugars such as for example maltodextrin as excipients, thereby allowing for additional flexibility in the development of suitable compositions.

In particular, preferred pharmaceutically acceptable acid addition salts of the present invention are non-hygroscopic, even more preferably crystalline and non-hygroscopic. In addition to avoiding the formation of impurities by slowing down the Maillard reaction, such preferred pharmaceutically acceptable acid addition salts possess improved chemical stability, in particular improved chemical stability upon contact with moisture.

### DETAILED DESCRIPTION OF THE INVENTION

The pharmaceutical composition of the present invention comprises omarigliptin and at least one pharmaceutically acceptable excipient, wherein the omarigliptin is in the form of a pharmaceutically acceptable acid addition salt.

Pharmaceutically acceptable acid addition salts of omarigliptin include, without limitation, the hydrochloride, nitrate, phosphate, sulfate, acetate, benzoate, citrate, malate, maleate, mesylate, besylate, succinate, tartrate, oxalate, lactate, fumarate and mandelate salt. Preferred pharmaceutically acceptable acid addition salts of omarigliptin are the hydrochloride, nitrate, phosphate, fumarate and mandelate salt. A particularly preferred pharmaceutically acceptable acid addition salt of omarigliptin is the fumarate salt. Another particularly preferred pharmaceutically acceptable acid addition salt of omarigliptin is the mandelate salt (including the omarigliptin R-mandelate, omarigliptin S-mandelate and omarigliptin RS-mandelate). Another particularly preferred pharmaceutically acceptable acid addition salt of omarigliptin is the hydrochloride salt. Omarigliptin can be prepared as described in WO 2010/056708. The pharmaceutically acceptable acid addition salts, including the pharmaceutically acceptable crystalline acid addition salts can be prepared as described in patent application number EP15201150.8.

Preferably, the pharmaceutically acceptable omarigliptin acid addition salts of the present invention is non-hygroscopic, i.e. show a water uptake of at most 2.0 weight%, preferably of at most 1.4 weight%, preferably of at most 1.0 weight%, preferably of at most 0.8 weight%, preferably of at most 0.7%, based on the weight of the omariglipitin acid addition salt, when measured with gravimetric moisture sorption at a relative humidity in the range of from 0 to 95% and a temperature of 25.0 ± 0.1 °C.

The pharmaceutically acceptable acid addition salts of omarigliptin of the present invention can be in amorphous form, crystalline form or in a mixture of crystalline and amorphous forms. Preferably, the pharmaceutically acceptable acid addition salts of omarigliptin of the present invention are in crystalline form. One preferred crystalline acid addition salt of omarigliptin is crystalline omarigliptin hydrochloride characterized by having a powder X-ray diffractogram comprising reflections at 2-Theta angles of (18.7 ± 0.2)°, (21.0 ± 0.2)° and (24.2 ± 0.2)°, when measured with Cu-Kalpha_{1,2} radiation at a wavelength of 0.15419 nm and a temperature in the range of from 20 to 30 °C. Another preferred crystalline acid addition salt of omarigliptin is crystalline omarigliptin fumarate characterized by having a powder X-ray diffractogram comprising reflections at 2-Theta angles selected from the group of (13.8 ± 0.2)°, (19.7 ± 0.2)° and (21.2 ± 0.2)°, when measured with Cu-Kalpha1,2 radiation at a wavelength of 0.15419 nm and a temperature in the range of from 20 to 30 °C. Another preferred crystalline acid addition salt of omarigliptin is crystalline omarigliptin (R)-mandelate characterized by having a powder X-ray diffractogram comprising reflections at 2-Theta angles selected from the group of (6.3 ± 0.2)°, (17.9 ± 0.2)° and (21.9 ± 0.2)°, when measured with Cu-Kalpha1,2 radiation at a wavelength of 0.15419 nm and a temperature in the range of from 20 to 30 °C. Another preferred crystalline acid addition salt of omarigliptin is crystalline omarigliptin (S)-mandelate characterized by having a powder X-ray diffractogram comprising reflections at 2-Theta angles selected from the group of (16.3 ± 0.2)°, (18.6 ± 0.2)° and (20.7 ± 0.2)°, when measured with Cu-Kalpha1,2 radiation at a wavelength of 0.15419 nm and a temperature in the range of from 20 to 30 °C. Another preferred crystalline acid addition salt of omarigliptin is crystalline omarigliptin (R, S)-mandelate characterized by having a powder X-ray diffractogram comprising reflections at 2-Theta angles selected from the group of (6.3 ± 0.2)°, (12.7 ± 0.2)° and (18.6 ± 0.2)°, when measured with Cu-Kalpha1,2 radiation at a wavelength of 0.15419 nm and a temperature in the range of from 20 to 30 °C. A particularly preferred crystalline acid addition salt of omarigliptin is crystalline omarigliptin hydrochloride characterized by having a powder X-ray diffractogram comprising reflections at 2-Theta angles of (18.7 ± 0.2)°, (21.0 ± 0.2)° and (24.2 ± 0.2)°, when measured with Cu-Kalpha_{1,2} radiation at a wavelength of 0.15419 nm and a temperature in the range of from 20 to 30 °C.

Regarding the at least one pharmaceutically acceptable excipient, no particular limitation exists provided suitable compositions are obtained. Preferably, the at least one pharmaceutically acceptable excipient comprises neutral excipients.

Neutral excipients are pharmaceutically acceptable excipients which do not have an ionic charge. Examples are diluents, such as microcrystalline cellulose and mannitol.

Thus, in a preferred embodiment of the present invention, the pharmaceutical composition comprises a pharmaceutically acceptable acid addition salt of omarigliptin and at least one pharmaceutically acceptable excipient, wherein at least one pharmaceutically acceptable excipient comprises neutral excipients, wherein the neutral excipients are present in an amount of at least 75% by weight based on the pharmaceutical composition and wherein the neutral excipients comprise at least one non-reducible sugar diluent. More preferably, the at least one pharmaceutically acceptable excipient comprises neutral excipients wherein the neutral excipients are present in an amount of at least 80% by weight based on the pharmaceutical composition, wherein the neutral excipients comprise at least one non-reducible sugar diluent. Even more preferably, the neutral excipients are present in an amount of at least 84% by weight based on the pharmaceutical composition.

In another preferred embodiment of the present invention, the pharmaceutical composition comprises a pharmaceutically acceptable acid addition salt of omarigliptin and at least one pharmaceutically acceptable excipient, wherein the at least one pharmaceutically acceptable excipient comprises neutral excipients, wherein the neutral excipients are present in an amount of less than 75% by weight, based on the pharmaceutical composition. Preferably, the neutral excipients are present in an amount of less than 70% by weight, preferably in an amount of less than 65% by weight, based on the pharmaceutical composition. With regard to the nature of the neutral excipients, no limitation exists provided suitable compositions are obtained. Preferably, the neutral excipients comprise microcrystalline cellulose, mannitol or a mixture of microcrystalline cellulose and mannitol.

The pharmaceutical composition according to the present invention, especially the pharmaceutical composition of any of the preferred embodiments described above, may additionally comprise one or more non-neutral excipients. Non-neutral excipients are pharmaceutically acceptable excipients which have an ionic charge, such as salts. The non-neutral excipients include, for example, disintegrants and lubricants. Thus, the at least one non-neutral excipient may comprise a disintegrant, a lubricant or a disintegrant and a lubricant.

An exemplified non-neutral disintegrant is croscarmellose sodium. Regarding the amount of non-neutral disintegrant, no limitations exist provided suitable compositions are obtained. The non-neutral disintegrant is preferably present in the pharmaceutical composition in an amount of 1 to 15% by weight.

An exemplified non-neutral lubricant is magnesium stearate. Regarding the amount of non-neutral lubricant, no limitations exist provided suitable compositions are obtained. The non-neutral lubricant is preferably present in the pharmaceutical composition in an amount of 1 to 3% by weight, more preferably in an amount of 1.5 to 3% by weight.

Preferably, the combined amount of neutral excipients and non-neutral excipients is less than 85% by weight, preferably less than 80% by weight, more preferably less than 75% by weight, even more preferably less than 70% by weight, based on the pharmaceutical composition.

In another preferred embodiment of the present invention, the pharmaceutical composition comprises a pharmaceutically acceptable acid addition salt of omarigliptin and at least one pharmaceutically acceptable excipient, wherein the at least one pharmaceutically acceptable excipient comprises a diluent, wherein the diluent comprises a reducing sugar, and wherein the reducing sugar is maltodextrin.

Maltodextrin is a saccharide mixture of polymers that consist of D-glucose units connected in chains of variable length. Maltodextrin is prepared by partial hydrolysis of starch.

Maltodextrin is a reducible sugar, it gives a positive reaction in the Fehling test, i.e. maltodextrin reduces Fehling's solution.

Maltodextrin usually has a dextrose equivalent (DE) of 1 to 20. Preferably, maltodextrin having a dextrose equivalent of 3 to 20, more preferably maltodextrin having a dextrose equivalent of 5 to 10, and even more preferably maltodextrin having a dextrose equivalent of 3 to 5 is used. The dextrose equivalent is a measure of the extent of polymer hydrolysis and is expressed in grams of D-glucose per 100 g of the dry substance. Preferably, the maltodextrin is the only reducing sugar present in the pharmaceutical composition. Also preferably, the pharmaceutical composition is free of mannitol or free of microcrystalline cellulose. More preferably, the pharmaceutical composition is free of mannitol and free of microcrystalline cellulose.

Regarding the amount of maltodextrin present in the composition, no limitation exists provided suitable compositions are obtained. Preferably, the maltodextrin is present in the pharmaceutical composition in an amount of 60 to 99% by weight, preferably 60 to 90% by weight, more preferably 60 to 80% by weight, even more preferably 60 to 75% by weight, based on the pharmaceutical composition.

The pharmaceutical composition comprising maltodextrin according to the preferred embodiment described above may additionally comprise a disintegrant, a lubricant or a disintegrant and a lubricant.

An exemplified disintegrant is croscarmellose sodium. Regarding the amount of disintegrant, no limitations exist provided suitable compositions are obtained. The disintegrant is preferably present in the pharmaceutical composition in an amount of 1 to 15% by weight.

An exemplified lubricant is magnesium stearate. Regarding the amount of lubricant, no limitations exist provided suitable compositions are obtained. The lubricant is preferably present in the pharmaceutical composition in an amount of 1 to 3% by weight, more preferably in an amount of 1.5 to 3% by weight.

Preferably, the pharmaceutical composition comprising maltodextrin according to the preferred embodiment described above comprises a disintegrant and a lubricant, wherein the disintegrant is croscarmellose sodium and the lubricant is magnesium stearate.

In another preferred embodiment of the present invention, the pharmaceutical composition comprises a pharmaceutically acceptable acid addition salt of omarigliptin and at least one pharmaceutically acceptable excipient, wherein the at least one pharmaceutically acceptable excipient comprises a diluent, wherein the diluent comprises a reducing sugar, and wherein the reducing sugar is lactose.

The pharmaceutical composition comprising lactose according to the preferred embodiment described above may additionally comprise a binder, a disintegrant and/or a lubricant, preferably a binder, a disintegrant and a lubricant.

An exemplified binder is povidone (polyvinylpyrrolidone, such as Kollidon 30). Regarding the amount of the binder, no limitations exist provided suitable compositions are obtained. The binder is preferably present in the pharmaceutical composition in an amount of 1 to 15% by weight.

An exemplified disintegrant is crospovidone (crosslinked polyvinylpyrrolidone, such as Kollidon CL). Regarding the amount of the disintegrant, no limitations exist provided suitable compositions are obtained. The disintegrant is preferably present in the pharmaceutical composition in an amount of 1 to 15% by weight.

An exemplified lubricant is magnesium stearate. Regarding the amount of the lubricant, no limitations exist provided suitable compositions are obtained. The lubricant is preferably present in the pharmaceutical composition in an amount of 1 to 3% by weight, more preferably in an amount of 1.5 to 3% by weight.

Preferably, the pharmaceutical composition comprising lactose according to the preferred embodiment described above comprises a binder, a disintegrant and a lubricant, wherein the binder is povidone, the disintegrant is crospovidone, and the lubricant is magnesium stearate.

In another preferred embodiment, the pharmaceutical composition of the present invention may comprise a glidant. The glidant is, for example, silicon dioxide. The glidant can be present in the pharmaceutical composition of the present invention in an amount of 0.2 to 2% by weight, preferably in an amount of 0.5 to 1 % by weight.

In any of the embodiments described above, the omarigliptin acid addition salt is present in an amount of 12.5 mg or 25 mg, calculated as a free base.

The pharmaceutical composition according to the present invention is preferably a solid oral dosage form, more preferably a tablet, a capsule or a powder. The tablet may be an uncoated tablet or a coated tablet. The uncoated tablet may be a tablet to be swallowed or a chewable tablet. In a particularly preferred embodiment, the pharmaceutical composition according to the present invention is a coated tablet. The tablet may have a round, oval or biconvex shape.

The coating preferably comprises hydroxypropyl methylcellulose (HPMC), hydroxypropyl cellulose (HPC) and titanium dioxide. The coating may additionally comprise talc. Optionally, a colorant, for example iron oxide red, iron oxide yellow, or an aluminum lake, may be present in the coating.

Preferably, the pharmaceutical composition of the present invention is obtainable by a process comprising a direct compression step.

In particular, the tablets can be manufactured by first mixing the excipients, except the magnesium stearate, in a suitable mixer. Optionally, the excipients are passed through a sieve, for example, through a 1.0 mm sieve, before mixing. Thereafter, the pharmaceutically acceptable acid addition salt of omarigliptin is added and the resulting mixture is mixed in a suitable mixer. Thereafter the magnesium stearate is added to the mixture. The thus obtained mixture is then blended in a suitable mixer, for example a Turbula mixer, for 1 to 5 minutes, preferably for about 3 minutes, and then compressed into tablets on a suitable tablet press, for example, a Korsch tablet press.

The tablets can be coated in a suitable coating device, for example, a pan coater or a fluid bed coating device.

The capsules can be prepared by mixing a pharmaceutically acceptable acid addition salt of omarigliptin with one or more excipients, optionally sieving the mixture, and filling the mixture into capsules, for example into hard gelatine capsules.

The powder can be prepared by mixing a pharmaceutically acceptable acid addition salt of omarigliptin with one or more excipients, optionally sieving the mixture, and filling the mixture into sachets.

The pharmaceutical composition of the present invention can be used in the treatment of metabolic diseases, in particular in the treatment of diabetes mellitus type 2.

The present invention is further illustrated by the following embodiments and combinations of embodiments as indicated by the respective dependencies and references.
1. A pharmaceutical composition comprising omarigliptin and at least one pharmaceutically acceptable excipient, wherein the omarigliptin is in the form of a pharmaceutically acceptable acid addition salt.
2. The pharmaceutical composition according to embodiment 1, wherein the pharmaceutically acceptable acid addition salt is selected from the group consisting of omarigliptin hydrochloride, omarigliptin sulfate, omarigliptin acetate, omarigliptin benzoate, omarigliptin citrate, omarigliptin malate, omarigliptin maleate, omarigliptin mesylate, omarigliptin besylate, omarigliptin succinate, omarigliptin tartrate, omarigliptin oxalate, omarigliptin lactate, omarigliptin mandelate, omarigliptin fumarate, omarigliptin nitrate and omarigliptin phosphate.
3. The pharmaceutical composition according to embodiments 1 or 2, wherein the pharmaceutically acceptable acid addition salt is omarigliptin hydrochloride.
4. The pharmaceutical composition according to any of embodiments 1 to 3, wherein the omarigliptin acid addition salt shows a water uptake of at most 2.0 weight%, preferably of at most 1.4 weight%, preferably of at most 1.0 weight%, preferably of at most 0.8 weight%, preferably of at most 0.7%, based on the weight of the omariglipitin acid addition salt, when measured with gravimetric moisture sorption at a relative humidity in the range of from 0 to 95% and a temperature of 25.0 ± 0.1 °C.
5. The pharmaceutical composition according to any of embodiments 1 to 4, wherein the omarigliptin acid addition salt is in amorphous form, crystalline form or in a mixture of crystalline and amorphous forms.
6. The pharmaceutical composition according to embodiment 3, wherein the omarigliptin acid addition salt is crystalline omarigliptin hydrochloride characterized by having a powder X-ray diffractogram comprising reflections at 2-Theta angles of (18.7 ± 0.2)°, (21.0 ± 0.2)° and (24.2 ± 0.2)°, when measured with Cu-Kalpha_{1,2} radiation at a wavelength of 0.15419 nm and a temperature in the range of from 20 to 30 °C.
7. The pharmaceutical composition according to any of embodiments 1 to 6, wherein the at least one pharmaceutically acceptable excipient comprises neutral excipients.
8. The pharmaceutical composition according to any of embodiments 1 to 6, wherein the at least one pharmaceutically acceptable excipient comprises neutral excipients and wherein the neutral excipients are present in an amount of at least 75% by weight based on the pharmaceutical composition, wherein the neutral excipients comprise at least one non-reducible sugar diluent.
9. The pharmaceutical composition according to embodiment 8, wherein the amount of neutral excipients comprises at least 80%, preferably at least 84% by weight based on the pharmaceutical composition.
10. The pharmaceutical composition according to any of embodiments 1 to 7, wherein the at least one pharmaceutically acceptable excipient comprises neutral excipients, wherein the neutral excipients are present in an amount of less than 75% by weight, based on the pharmaceutical composition.
11. The pharmaceutical composition according to embodiment 10, wherein the neutral excipients are present in an amount of less than 70% by weight, preferably in an amount of less than 65% by weight, based on the pharmaceutical composition.
12. The pharmaceutical composition according to any of embodiments 1 to 11, wherein the neutral excipients comprise microcrystalline cellulose.
13. The pharmaceutical composition according to any of embodiments 1 to 11, wherein the neutral excipients comprise mannitol.
14. The pharmaceutical composition according to any of embodiments 1 to 11, wherein the neutral excipients comprise microcrystalline cellulose and mannitol.
15. The pharmaceutical composition according to any of embodiments 1 to 14 further comprising at least one non-neutral excipient.
16. The pharmaceutical composition according to embodiment 15, wherein the at least one non-neutral excipient comprises a disintegrant and/or a lubricant.
17. The pharmaceutical composition according to embodiment 16, wherein the disintegrant is croscarmellose sodium.
18. The pharmaceutical composition according to embodiment 16, wherein the lubricant is magnesium stearate.
19. The pharmaceutical composition according to any of embodiments 10 to 18, wherein the combined amount of neutral excipients and non-neutral excipients is less than 85% by weight, preferably less than 80% by weight, more preferably less than 75% by weight, even more preferably less than 70% by weight, based on the pharmaceutical composition.
20. The pharmaceutical composition according to any of embodiments 1 to 6, wherein the at least one pharmaceutically acceptable excipient comprises a diluent, wherein the diluent comprises a reducing sugar, and wherein the reducing sugar is maltodextrin.
21. The pharmaceutical composition according to embodiment 20, wherein the maltodextrin has a dextrose equivalent of 1 to 20, preferably 3 to 20, more preferably 5 to 10.
22. The pharmaceutical composition according to any of embodiments 20 or 21, wherein the maltodextrin is the only reducing sugar present in the pharmaceutical composition.
23. The pharmaceutical composition according to any of embodiments 20 to 22, wherein the maltodextrin is the only diluent present in the pharmaceutical composition.
24. The pharmaceutical composition according to any of embodiments 20 to 23, wherein the pharmaceutical composition is free of mannitol.
25. The pharmaceutical composition according to any of embodiments 20 to 24, wherein the pharmaceutical composition is free of microcrystalline cellulose.
26. The pharmaceutical composition according to any of embodiments 20 to 25, wherein the pharmaceutical composition is free of mannitol and free of microcrystalline cellulose.
27. The pharmaceutical composition according to any of embodiments 20 to 26, wherein the maltodextrin is present in the pharmaceutical composition in an amount of 60 to 99% by weight, preferably 60 to 90% by weight, more preferably 60 to 80% by weight, even more preferably 60 to 75% by weight, based on the pharmaceutical composition.
28. The pharmaceutical composition according to any of embodiments 20 to 27 comprising additionally a disintegrant and/or a lubricant.
29. The pharmaceutical composition according to embodiment 28, wherein the disintegrant is croscarmellose sodium.
30. The pharmaceutical composition according to embodiment 28, wherein the lubricant is magnesium stearate.
31. The pharmaceutical composition according to any of embodiments 28 to 30, wherein the disintegrant is croscarmellose sodium and the lubricant is magnesium stearate.
32. The pharmaceutical composition according to any of embodiments 1 to 31, wherein the omarigliptin acid addition salt is present in an amount of 12.5 mg or 25 mg, calculated as a free base.
33. The pharmaceutical composition according to any of embodiments 1 to 32, which is a solid oral dosage form, preferably a tablet, a capsule, a granulate, pellets or a powder.
34. The pharmaceutical composition according to embodiment 33 which is a tablet, preferably an uncoated tablet or a coated tablet.
35. The pharmaceutical composition according to embodiment 34 obtainable by a process comprising a direct compression step.
36. The pharmaceutical composition according to any of embodiments 1 to 35 for use in the treatment of diabetes mellitus type 2.

### EXAMPLES

The following examples illustrate the present invention and are not intended to limit the scope of the invention set forth in the claims appended hereto.

**Example 1 - Tablet formulation**

| **Component** | **Amount** | **Amount** |
|---|---|---|
| | **(mg)** | **(% by weight)** |
| Omarigliptin HCl¹ | 27.2 | 17 |
| Ludipress^{®2} | 128.0 | 80 |
| Magnesium stearate | 4.8 | 3 |
| | | |
| Sum components | 160 | 100 |
| Sum excipients | 132.8 | 83 |

| | | |
|---|---|---|
| ¹Omarigliptin HCl was used in crystalline form as described above. ²Ludipress is the brand name of an excipient mixture marketed by BASF SE which comprises 93% by weight Lactose, 3.5% by weight Kollidon 30 (polyvinylpyrrolidone) and 3.5% by weight Kollidon CL (cross-linked polyvinylpyrrolidone). | | |

All components in the amounts mentioned in the above table, except the magnesium stearate, were mixed and passed through a 1.0 mm sieve. The magnesium stearate in the amount mentioned in the above table was then added to the mixture. The thus obtained mixture was blended in a Turbula mixer for 3 minutes and then compressed into round tablets having a diameter of 8 mm in a Korsch XP1, 8 OR tablet press.

**Example 2 - Tablet formulation**

| **Component** | **Amount** | **Amount** |
|---|---|---|
| | **(mg)** | **(% by weight)** |
| Omarigliptin HCl | 27.2 | 16.77 |
| Maltodextrin | 111 | 68.43 |
| Croscarmellose sodium | 20 | 12.33 |
| Magnesium stearate | 4 | 2.47 |
| | | |
| Sum components | 162.2 | 100 |
| Sum excipients | 135 | 83.23 |

The excipients in the amounts mentioned in the above table, except the magnesium stearate, are first passed through a 1.0 mm sieve and then mixed. The omarigliptin hydrochloride in the amount mentioned in the above table is blended into the mixture. Then, the magnesium stearate in the amount mentioned in the above table is added to the mixture. The thus obtained mixture is blended in a Turbula mixer for 3 minutes and then compressed into round tablets having a diameter of 8 mm in a Korsch XP1, 8 OR tablet press.

**Example 3 - Coated tablet formulation**

| **Component** | **Amount** | **Amount** |
|---|---|---|
| | **(mg)** | **(% by weight)** |
| Omarigliptin HCl | 27.2 | 32.85 |
| Maltodextrin | 52 | 62.80 |
| Croscarmellose sodium | 1.6 | 1.93 |
| Magnesium stearate | 2 | 2.42 |
| | | |
| Sum components | 80.6 | 100 |
| Sum excipients | 55.6 | 67.15 |

The excipients in the amounts mentioned in the above table, except the magnesium stearate, are first passed through a 1.0 mm sieve and then mixed. The omarigliptin hydrochloride in the amount mentioned in the above table is blended into the mixture. Then, the magnesium stearate in the amount mentioned in the above table is added to the mixture. The thus obtained mixture is blended in a Turbula mixer for 3 minutes and then compressed into round tablets having a diameter of 6 mm in a Korsch XP1, 8 OR tablet press.

The tablets are coated in a Glatt Minicoater with an aqueous solution of 4.8 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

## Claims

1. A pharmaceutical composition comprising omarigliptin and at least one pharmaceutically acceptable excipient, wherein the omarigliptin is in the form of a pharmaceutically acceptable acid addition salt.

2. The pharmaceutical composition according to claim 1, wherein the pharmaceutically acceptable acid addition salt is selected from the group consisting of omarigliptin hydrochloride, omarigliptin sulfate, omarigliptin acetate, omarigliptin benzoate, omarigliptin citrate, omarigliptin malate, omarigliptin maleate, omarigliptin mesylate, omarigliptin besylate, omarigliptin succinate, omarigliptin tartrate, omarigliptin oxalate, omarigliptin lactate, omarigliptin mandelate, omarigliptin fumarate, omarigliptin nitrate and omarigliptin phosphate.

3. The pharmaceutical composition according to claims 1 or 2, wherein the pharmaceutically acceptable acid addition salt is omarigliptin hydrochloride.

4. The pharmaceutical composition according to any of claims 1 to 3, wherein the omarigliptin acid addition salt shows a water uptake of at most 2.0 weight%, preferably of at most 1.4 weight%, preferably of at most 1.0 weight%, preferably of at most 0.8 weight%, preferably of at most 0.7%, based on the weight of the omariglipitin acid addition salt, when measured with gravimetric moisture sorption at a relative humidity in the range of from 0 to 95% and a temperature of 25.0 ± 0.1 °C.

5. The pharmaceutical composition according to any of claims 1 to 4, wherein the omarigliptin acid addition salt is in amorphous form, crystalline form or in a mixture of crystalline and amorphous forms.

6. The pharmaceutical composition according to claim 3, wherein the omarigliptin acid addition salt is crystalline omarigliptin hydrochloride **characterized by** having a powder X-ray diffractogram comprising reflections at 2-Theta angles of (18.7 ± 0.2)°, (21.0 ± 0.2)° and (24.2 ± 0.2)°, when measured with Cu-Kalpha_{1,2} radiation at a wavelength of 0.15419 nm and a temperature in the range of from 20 to 30 °C.

7. The pharmaceutical composition according to any of claims 1 to 6, wherein the at least one pharmaceutically acceptable excipient comprises neutral excipients, preferably wherein the neutral excipients are present in an amount of at least 75% by weight, preferably of at least 80% by weight, more preferably of at least 84% by weight, based on the pharmaceutical composition, wherein the neutral excipients comprise at least one non-reducible sugar diluent.

8. The pharmaceutical composition according to any of claims 1 to 6, wherein the at least one pharmaceutically acceptable excipient comprises neutral excipients, wherein the neutral excipients are present in an amount of less than 75% by weight, preferably in an amount of less than 70% by weight, more preferably in an amount of less than 65% by weight, based on the pharmaceutical composition.

9. The pharmaceutical composition according to any of claims 1 to 8, wherein the neutral excipients comprise microcrystalline cellulose, mannitol or a mixture of microcrystalline cellulose and mannitol.

10. The pharmaceutical composition according to any of claims 1 to 9 further comprising at least one non-neutral excipient, peferably wherein the at least one non-neutral excipient comprises a disintegrant and/or a lubricant.

11. The pharmaceutical composition according to any of claims 1 to 6, wherein the at least one pharmaceutically acceptable excipient comprises a diluent, wherein the diluent comprises a reducing sugar, and wherein the reducing sugar is maltodextrin, preferably wherein the maltodextrin has a dextrose equivalent of 1 to 20, preferably 3 to 20, more preferably 5 to 10.

12. The pharmaceutical composition according to claim 11, wherein the maltodextrin is the only reducing sugar present in the pharmaceutical composition.

13. The pharmaceutical composition according to any of claims 11 to 12, wherein the pharmaceutical composition is free of mannitol, free of microcrystalline cellulose or free of mannitol and free of microcrystalline cellulose.

14. The pharmaceutical composition according to any of claims 11 to 13, wherein the maltodextrin is present in the pharmaceutical composition in an amount of 60 to 99% by weight, preferably 60 to 90% by weight, more preferably 60 to 80% by weight, even more preferably 60 to 75% by weight, based on the pharmaceutical composition.

15. The pharmaceutical composition according to any of claims 1 to 14, wherein the omarigliptin acid addition salt is present in an amount of 12.5 mg or 25 mg, calculated as a free base.
